# EUROPEAN PATENT APPLICATION

(11) **EP 1 780 205 A1**
(43) Date of publication of application: **02.05.2007**
(21) Application number: 05746074.3
(22) Date of filing: 02.06.2005
(51) Int. Cl.: C07D 307/14

(54) **METHOD FOR PRODUCING 3-AMINOMETHYLTETRAHYDROFURAN DERIVATIVE**

(30) Priority: 14.06.2004 JP 2004175322
(71) Applicant: Mitsui Chemicals, Inc., Tokyo 105-7117 (JP)
(72) Inventor: INOUE, Yoshihisa, Mitsui Chemicals, Inc., Sodegaura-shi, Chiba 2990265 (JP); FUNAKI, Setsuko, Mitsui Chemicals, Inc., Sodegaura-shi, Chiba 2990265 (JP); HAMADA, Tetsuya, Mitsui Chemicals, Inc., Sodegaura-shi, Chiba 2990265 (JP); YAMAMOTO, Yoshihiro, Mitsui Chemicals, Inc., Sodegaura-shi, Chiba 2990265 (JP); HARA, Isao, Mitsui Chemicals, Inc., Sodegaura-shi, Chiba 2990265 (JP); HAYASHI, Takaomi, Mitsui Chemicals, Inc., Sodegaura-shi, Chiba 2990265 (JP); KONNO, Miyuki, Mitsui Chemicals, Inc., Sodegaura-shi, Chiba 2990265 (JP); AOKI, Shinobu, Mitsui Chemicals, Inc., Sodegaura-shi, Chiba 2990265 (JP)
(74) Representative: Stuart, Ian Alexander
(86) International application number: PCT/JP2005/010171
(87) International publication number: WO 2005/121111

(57) **Abstract**

An object of the present invention is to provide a process for producing a 3-cyanotetrahydrofuran derivative in a high yield from inexpensive industrial materials.

According to the present invention, a 3-aminomethyltetrahydrofuran derivative is produced by preparing a 3-cyanotetrahydrofuran derivative in a high yield from an inexpensive and industrially easily available malic acid derivative, and reducing the cyano group of the 3-cyanotetrahydrofuran derivative.

## Description

### TECHNICAL FIELD

The present invention relates to a process for producing a 3-aminomethyltetrahydrofuran derivative. More specifically, the invention relates to a process for producing a 3-aminomethyltetrahydrofuran derivative reducing a 3-cyanotetrahydrofuran derivative. The 3-aminomethyltetrahydrofuran derivative is useful as an intermediate for medicines, agrochemicals, etc.

### BACKGROUND ART

A (tetrahydro-3-furanyl)methylamine derivative having a structure represented by the following formula (1) exhibits an extremely high insecticidal activity, and further has low toxicity and an extremely excellent performance as an active ingredient of agrochemicals (see Patent Document 1). However, a process for producing a 3-aminomethyltetrahydrofuran derivative as raw materials thereof is little known and only the following two methods are known. This is on the ground that the synthesis of a tetrahydrofuran compound having a substituent at the 3-position is extremely difficult, while a tetrahydrofuran compound having a substituent at the 2-position can be easily derived from the substitution reaction on the furan ring.

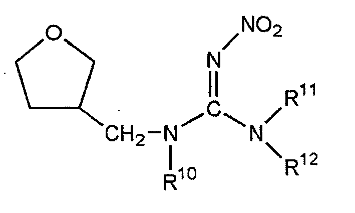

wherein R¹⁰, R¹¹ and R¹² represent a hydrogen atom or a lower alkyl group.
As one process for producing a 3-aminomethyltetrahydrofuran derivative, a method which includes carrying out a reductive amination using tetrahydrofuran-3-carboxyaldehyde as a raw material in an aqueous ammonia solution in the presence of hydrogen, is known (see Patent Document 2). However, this method cannot be said to be an industrially sufficiently advantageous method in that extremely expensive raw materials has to be used, for example, expensive 2-butene-1, 4-diol has to be subjected to cyclization and then further hydroformylation using an expensive rhodium catalyst in order to obtain tetrahydrofuran-3-carboxyaldehyde being the raw material.

Further, as another method, for example, Patent Document 1 discloses a method in which 3-(tetrahydrofuryl)methylhalide or 3-(tetrahydrofuryl)methylsulfonate is derived from 3-hydroxymethyltetrahydrofuran as raw materials and is reacted with potassium phthalimide to carry out hydrolysis or hydrazinolysis. However, this method is also not industrially advantageous in that 2-hydroxymethyl-1,4-butanediol being raw materials of 3-hydroxymethyltetrahydrofuran is expensive, by-products derived from phthalimide are formed in large quantities and the highly hazardous hydrazine is used.

Furthermore, for a hydrogenation method of a 3-cyanotetrahydrofuran derivative, there are not many synthesis examples of effectively obtaining a 3-cyanotetrahydrofuran derivative and thus examples of a process for producing a 3-aminomethyltetrahydrofuran derivative using it as raw materials are not known. A method of obtaining amines by reducing a cyano group is generally known, but the preferred method thereof is largely varied depending on a substrate. For the reduction reaction of a cyano compound having a tetrahydrofuran ring, a 3-cyanotetrahydrofuran derivative and a 2-cyanotetrahydrofuran derivative as its isomer are not completely known and thus the preferred method thereof is also not completely known.

Further, as a process for producing a 3-cyanotetrahydrofuran derivative, only two examples consisting of a method of ultraviolet irradiation of tetrahydrofuran and chlorocyan in the presence of sodium bicarbonate (see Patent Document 3) and a method of reacting ethylene oxide and acrylonitrile in the presence of a catalyst (see Patent Document 4) are known. The former is low in both yield and selectivity and the latter is high in selectivity but no description is given on the yield, and therefore, any process cannot be said to be effective as a process for producing a 3-cyanotetrahydrofuran derivative. A process for producing a 3-cyanotetrahydrofuran derivative from a 3-halogenated or 3-alkylsulfonated or 3-arylsulfonated tetrahydrofuran derivative, which is a process according to the present invention, is not known.

Furthermore, a process for producing a 3-halogenated or 3-alkylsulfonated or 3-arylsulfonated tetrahydrofuran derivative from a 3-hydroxytetrahydrofuran derivative, which is a process according to the present invention, is also not known.
[Patent Document 1] Japanese Patent Application Laid-Open (Jp-A) No. 7-179448
[Patent Document 2] JP-A No. 9-110848
[Patent Document 3] German Patent No. 1234227
[Patent Document 4] JP-A No. 2000-264884

### DISCLOSURE OF THE INVENTION

Accordingly, an industrially sufficiently advantageous process for producing a 3-aminomethyltetrahydrofuran derivative has not been yet found and thus a process for effectively producing the derivative from inexpensive raw materials has been eagerly desired. An object of the present invention is to provide a process for producing a 3-aminomethyltetrahydrofuran derivative with high efficiency from inexpensive industrial materials.

The present inventors have intensively investigated in order to achieve the above object, and as a result, they have found a process for producing a 3-aminomethyltetrahydrofuran derivative by reducing the cyano group of a 3-cyanotetrahydrofuran derivative and further a process for producing a 3-cyanotetrahydrofuran derivative in a high yield from an inexpensive and industrially easily available malic acid derivative, and thus have completed the present invention.

That is, a first aspect of the present invention provides a process for producing a 3-aminomethyltetrahydrofuran derivative represented by the formula (2):

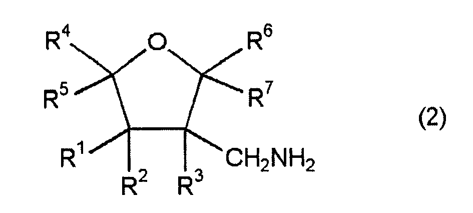

wherein R¹, R², R³, R⁴, R⁵, R⁶ and R⁷ may be the same or different from each other and each represent a hydrogen atom or a hydrocarbon group having 1 to 4 carbon atoms, which includes reducing the cyano group of a 3-cyanotetrahydrofuran derivative represented by the formula (1):

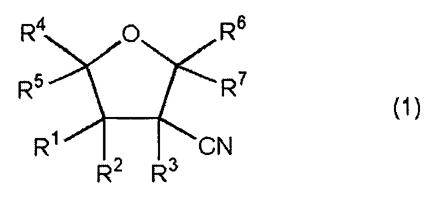

wherein R¹, R², R³, R⁴, R⁵, R⁶ and R⁷ may be the same or different from each other and each represent a hydrogen atom or a hydrocarbon group having 1 to 4 carbon atoms.

A second aspect of the present invention provides a process for producing a 3-cyanotetrahydrofuran derivative represented by the formula (1), which includes reacting a 3-halogenated or 3-alkylsulfonated or 3-arylsulfonated tetrahydrofuran derivative represented by the formula (3):

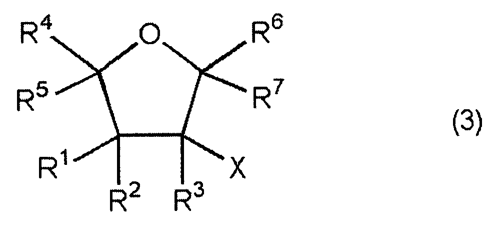

wherein R¹, R², R³, R⁴, R⁵, R⁶ and R⁷ may be the same or different from each other and each represent a hydrogen atom or a hydrocarbon group having 1 to 4 carbon atoms; and X represents a halogen atom or an alkylsulfonate group having 1 to 6 carbon atoms or arylsulfonate group having 6 to 12 carbon atoms, and an organic or inorganic cyano compound.

A third aspect of the present invention provides a process for producing a 3-aminomethyltetrahydrofuran derivative by the process according to the first aspect of the present invention, which includes using the 3-cyanotetrahydrofuran derivative obtained by the process according to the second aspect of the present invention.

A fourth aspect of the present invention provides a process for producing a 3-aminomethyltetrahydrofuran derivative by the process according to the third aspect of the present invention, which includes using a 3-halogenated or 3-alkylsulfonated or 3-arylsulfonated tetrahydrofuran derivative represented by the formula (3a):

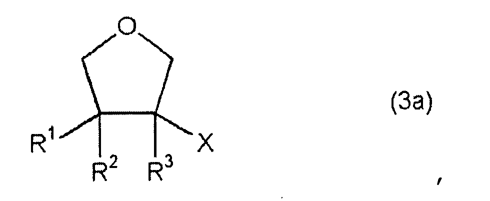

which produces from a malic acid derivative by three steps.

According to the present invention, the 3-aminomethyltetrahydrofuran derivative can be industrially advantageously produced from inexpensive raw materials compared to conventional processes.

### BEST MODE FOR CARRYING OUT THE INVENTION

Hereinafter, a process for producing a 3-aminomethyltetrahydrofuran derivative according to the present invention is specifically explained.

The 3-cyanotetrahydrofuran derivative according to the process of the invention is represented by the formula (1).

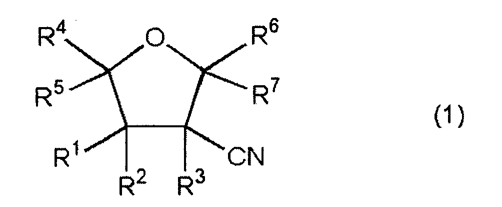

wherein all of R¹, R², R³, R⁴, R⁵, R⁶ and R⁷ may be the same or different from each other and each represent a hydrogen atom or a hydrocarbon group having 1 to 4 carbon atoms. More specific examples of the hydrocarbon group having 1 to 4 carbon atoms include a methyl group, an ethyl group, a n-propyl group, an isopropyl group, a n-butyl group, a 2-butyl group, an isobutyl group and t-butyl group. Specific examples of the 3-cyanotetrahydrofuran derivative include 3-cyanotetrahydrofuran substituted with a hydrocarbon group, such as 4-ethyl-3-cyanotetrahydrofuran and 3-methyl-3-cyanotetrahydrofuran, including 3-cyanotetrahydrofuran.

The process for producing a 3-cyanotetrahydrofuran derivative used in the present invention is not limited, but the 3-cyanotetrahydrofuran derivative can be more suitably produced by a process of cyanation of a 3-halogenated or 3-alkylsulfonated or 3-arylsulfonated tetrahydrofuran derivative.

In the present invention, the 3-aminomethyltetrahydrofuran derivative represented by the formula (2) is produced by reducing the cyano group of the 3-cyanotetrahydrofuran derivative represented by the formula (1).

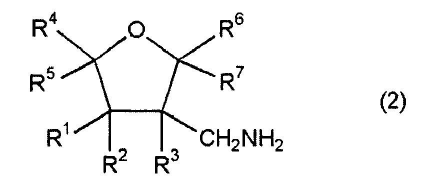

wherein R¹, R², R³, R⁴, R⁵, R⁶, R⁷ are as defined for R¹, R², R³, R⁴, R⁵, R⁶, R⁷ in the formula (1).
Specific examples of the 3-aminomethyltetrahydrofuran derivative include a 3-cyanotetrahydrofuran substituted with a hydrocarbon group, such as 4-ethyl-3-aminomethyltetrahydrofuran and 3-methyl-3-aminomethyltetrahydrofuran, including 3-aminomethyltetrahydrofuran. Since the 3-aminomethyltetrahydrofuran derivative obtained in the process of the present invention corresponds to the 3-cyanotetrahydrofuran derivative used, R¹, R², R³, R⁴, R⁵, R⁶ and R⁷ of each substituent in the 3-cyanotetrahydrofuran derivative used, R¹, R², R³, R⁴, R⁵, R⁶ and R⁷ of each substituent in the 3-aminomethyltetrahydrofuran derivative obtained are the same. For example, 3-aminomethyltetrahydrofuran is obtained when 3-cyanotetrahydrofuran is used and 3-methyl-3-aminomethyltetrahydrofuran is obtained when 3-methyl-3-cyanotetrahydrofuran is used.

Examples of the method of reducing the cyano group of the 3-cyanotetrahydrofuran derivative in the present invention include a method of reducing with a metal hydride and a method of reducing with hydrogen in the presence of a hydrogenation catalyst.

The specific examples of the metal hydride when reducing with the metal hydride include an aluminum hydride compound such as lithium aluminum hydride, lithium trimethoxyaluminum hydride, aluminum hydride and diisobutylaluminum hydride, and a boron hydride compound such as diborane, lithium borohydride and sodium borohydride. The amount used of the metal hydride when reducing by the metal hydride is usually in the range of 2 to 10 moles, preferably 3 to 6 moles per mole of a 3-cyanotetrahydrofuran derivative.

The hydrogenation catalyst when reducing with hydrogen in the presence of the hydrogenation catalyst may be any compound as long as it catalyzes the reaction in which the cyano group of the 3-cyanotetrahydrofuran derivative according to the present invention is reduced to an aminomethyl group by a molecular hydrogen, but usually at least one metal selected from Groups 7 to 13 of the Periodic Table of the Elements or a metal compound thereof is suitably used. More specifically, it includes a metal such as manganese, rhenium, iron, ruthenium, osmium, cobalt, rhodium, iridium, nickel, palladium, platinum, copper, silver, gold, zinc and aluminum or metal compounds thereof. These metal or metal compounds may be or may not be dissolved in a reaction liquid. Specific examples of these hydrogenation catalysts include an elemental metal such as rhodium metal powders and palladium metal powders, a Raney metal compound such as Raney nickel, Raney copper and Raney cobalt, a stabilized metal compound such as stabilized nickel, a metal-supported catalyst in which a metal such as rhenium, ruthenium, rhodium, palladium is supported on the inorganic support such as carbon black, activated carbon, alumina, silica gel, diatom earth, zeolite and magnesia, a metal oxide such as ruthenium oxide, palladium oxide, rhenium oxide and copper oxide, a complex metal-oxide such as copper oxide-chromium oxide, copper oxide-zinc oxide-aluminum oxide, complex compounds of metals of Groups 8 to 10 of the Periodic Table of the Elements, such as RuClH(CO) (PPh₃)₃, RuCl₂(PPh₃)₃, PdCl₂(PPh₃)₂. These catalysts may be used alone or in combination of two or more thereof. The amount of the hydrogenation catalyst to be used is in the range of usually 0.1 ppm by weight to 10% by weight, preferably 1 ppm by weight to 5% by weight relative to the 3-cyanotetrahydrofuran derivative when the catalyst used is dissolved in the reaction liquid, and is in the range of usually 0.1% by weight to 500% by weight, preferably 1% by weight to 200% by weight relative to the 3-cyanotetrahydrofuran derivative when the catalyst used is not dissolved in the reaction liquid.

Of these reduction methods, the method of reducing with hydrogen in the presence of the hydrogenation catalyst is preferred. The hydrogenation catalyst is preferably a metal of Group 9 or 10 of the Periodic Table of the Elements or a metal compound thereof, more preferably a metal such as cobalt or nickel or a metal compound thereof, and most preferably a metal such as cobalt or a metal compound thereof.

The method of reducing the 3-cyanotetrahydrofuran derivative of the present invention may be carried out without a solvent, but is usually carried out in the presence of the solvent. The suitable solvent to be used varies depending on the method of reducing the cyano group.

When it is reduced by the metal hydride, aliphatic or alicyclic hydrocarbons having 5 to 20 carbon atoms, such as n-hexane, n-pentane or cyclohexane, aromatic hydrocarbons having 6 to 20 carbon atoms such as benzene, toluene and ethylbenzene, aliphatic or aromatic halides having 1 to 20 carbon atoms such as chloroform, chlorobenzene and dichlorobenzene, and ethers having 2 to 20 carbon atoms such as diethyl ether, diphenyl ether, tetrahydrofuran and ethylene glycol dimethyl ether, are suitably used. Further, these solvents may be used in a mixture of two or more thereof. Of these solvents, ethers are particularly used.

As the solvent to be used when reducing with hydrogen in the presence of hydrogenation catalyst, for example, water, alcohols having 1 to 20 carbon atoms, such as methanol, ethanol, butanol, aliphatic or alicyclic hydrocarbons having 5 to 20 carbon atoms, such as n-hexane, n-pentane or cyclohexane, benzene, aromatic hydrocarbons having 6 to 20 carbon atoms, such as toluene and ethylbenzene, and ethers having 2 to 20 carbon atoms, such as diethyl ether, diphenyl ether, tetrahydrofuran and ethylene glycol dimethyl ether, are suitably used. Further, these solvents may be used in a mixture of two or more thereof. Of these solvents, water, alcohols and ethers are preferably used, and water is more preferably used.

The amount of the solvent used is not uniform over reaction conditions, but is in the range of usually 0.01 to 200 parts by weight, preferably 0.02 to 50 parts by weight, more preferably 0.05 to 2 parts by weight per part by weight of the 3-cyanotetrahydrofuran derivative.

In the method of reducing the 3-cyanotetrahydrofuran derivative of the present invention, when reducing the cyano group by hydrogen in the presence of the hydrogenation catalyst, it is preferable to carry out the reduction reaction in the presence of ammonia. The term "ammonia" as used in the present invention refers to aqueous ammonia, liquid ammonia or ammonia gas, but ammonia is more preferably aqueous ammonia. The amount of ammonia used is not particularly limited, but is in the range of usually 0.01 to 50 mols, preferably 0.1 to 20 mols, more preferably 0.3 to 5 moles relative to 1 mole of the 3-cyanotetrahydrofuran derivative.

The method of reducing the 3-cyanotetrahydrofuran derivative of the present invention is particularly preferably a method of reducing with hydrogen in the presence of ammonia and also in the presence of a metal of Group 9 or 10 of the Periodic Table of the Elements or a metal compound thereof, as a catalyst. In this case, the catalyst is more preferably a metal of nickel or cobalt or a metal compound thereof and ammonia is more preferably aqueous ammonia. Further, when the reaction is carried out in the presence of aqueous ammonia, it is preferably carried out in the presence of 0.05 to 2 parts by weight of aqueous ammonia relative to 1 part by weight of the 3-cyanotetrahydrofuran derivative.

In the method of reducing the 3-cyanotetrahydrofuran derivative of the present invention, all of R¹, R², R³, R⁴, R⁵, R⁶ and R⁷ are preferably a hydrogen atom.

The temperature and time in the reaction are not uniform over the kinds of the 3-cyanotetrahydrofuran derivative and the reduction method. When reducing with a metal hydride, the reaction temperature is in the range of usually -10 to 150°C, preferably 0 to 120°C, more preferably 10 to 100°C. When reducing with hydrogen in the presence of the hydrogenation catalyst, the reaction temperature is in the range of usually 0 to 250°C, preferably 50 to 200°C, more preferably 80 to 150°C. The reaction time is in the range of usually not more than 100 hours, preferably 0.01 to 50 hours.

The pressure in the reaction may be any of reduced pressure, normal pressure or pressurization, but the preferable mode varies depending on the reduction method to be performed. When reducing with the metal hydride, it is preferably carried out at normal pressure. When reducing with hydrogen in the presence of the hydrogenation catalyst, it is preferably carried out under hydrogen pressurization. The hydrogen pressure in pressurization is in the range of usually 0.01 to 25 MPaG, preferably 0.1 to 20 MPaG, more preferably 1 to 10 MPaG.

The reaction process of reducing the cyano group is not particularly limited and may be performed in any one of batch, semibatch and continuous flow modes.

When reducing with hydrogen in the presence of the hydrogenation catalyst, the catalyst used may be recovered by the conventional process for recovery of metals after completion of the reaction. For example, when the catalyst is dissolved in the reaction liquid, the catalyst can be recovered by contacting with a metal adsorbent such as an ion-exchange resin or by extracting with the solvent. When the catalyst is not dissolved in the reaction liquid, the catalyst can be recovered by a solid-liquid separation method such as filtration and centrifugal separation. These recovered catalysts can be repeatedly used as the hydrogenation catalyst. In that case, it may be reused after the catalyst deactivated or having reduced activity is subjected to a recycling operation or may be used by adding the fresh catalyst.

The 3-aminomethyltetrahydrofuran derivative formed by the method of reducing the 3-cyanotetrahydrofuran derivative of the present invention can be isolated according to the conventional separation method such as distillation.

Next, the process for producing the 3-cyanotetrahydrofuran derivative of the present invention is explained in detail in the following. The 3-cyanotetrahydro furan derivative represented by the formula (1) is prepared by reacting the 3-halogenated or 3-alkylsulfonated or 3-arylsulfonated tetrahydrofuran derivative represented by the formula (3) and an organic or inorganic cyano compound.

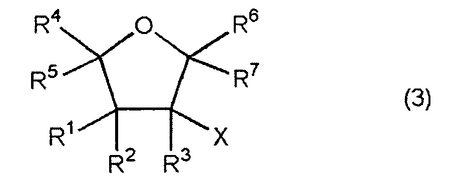

wherein R¹, R², R³, R⁴, R⁵, R⁶, R⁷ are as defined for R¹, R², R³, R⁴, R⁵, R⁶, R⁷ in the formula (1) ; and X represents a halogen atom or an alkylsulfonate group having 1 to 6 carbon atoms or an arylsulfonate group having 6 to 12 carbon atoms.
Specific examples of the halogen atom include a fluorine atom, a chlorine atom, a bromine atom and an iodine atom, specific examples of the alkylsulfonate group having 1 to 6 carbon atoms include a hydrocarbon alkylsulfonate group having 1 to 6 carbon atoms such as a methylsulfonate group, an ethylsulfonate group, and a halogen group-substituted alkylsulfonate group having 1 to 6 carbon atoms such as a trifluoromethylsulfonate group and a 1,1,1-trifluoroethylsulfonate group. Further, examples of the arylsulfonate group having 6 to 12 carbon atoms include a hydrocarbon arylsulfonate group having 6 to 12 carbon atoms such as a benzenesulfonate group and a p-toluenesulfonate group and a halogen group-substituted arylsulfonate group having 6 to 12 carbon atoms such as a p-trifluoromethyl benzenesulfonate group. More specific examples of the 3-halogenated or 3-alkylsulfonated or 3-arylsulfonated tetrahydrofuran derivative include 3-halogenated tetrahydrofuran such as 3-chlorotetrahydrofuran and 3-bromotetrahydrofuran, a hydrocarbon group-substituted 3-halogenated tetrahydrofuran such as 4-ethyl-3-chlorotetrahydrofuran and 3-methyl-3-iodotetrahydrofuran, 3-alkyl or arylsulfonated tetrahydrofuran such as 3-(p-toluenesulfonato)-tetrahydrofuran, 3-trifluoromethanesulfonatotetrahydrofuran and a hydrocarbon group-substituted 3-alkyl or arylsulfonated tetrahydrofuran such as 4-ethyl-3-benzenesulfonatotetrahydrofuran.

The process for producing the 3-halogenated or 3-alkylsulfonated or 3-arylsulfonated tetrahydrofuran derivative used in the process of the present invention is not limited, but it can be suitably produced by using a malic acid derivative described below as a starting material.

The organic or inorganic cyano compound used in the process of the present invention is an organic or inorganic cyano compound capable of converting a halogen group or alkyl or arylsulfonate group at the 3-position of the 3-halogenated or 3-alkylsulfonated or 3-arylsulfonated tetrahydrofuran derivative to the cyano group. Specific examples of the organic cyano compound include a hydrogen cyanide adduct of ketone or aldehyde having 1 to 20 carbon atoms, such as glycolonitrile and acetonecyanhydrin and a cyanated organic ammonium salt such as tetramethylammonium cyanide and triethylammonium cyanide. Examples of the inorganic cyano compound include ammonium cyanide, an alkali metal cyanide such as lithium cyanide, sodium cyanide and potassium cyanide, an alkaline earth metal cyanide such as magnesium cyanide, a transition metal cyanide of Groups 3 to 12 of the Periodic Table of the Elements, such as manganese cyanide, copper cyanide and cyanide ruthenium, including hydrogen cyanide. Of these organic or inorganic cyano compounds, the hydrogen cyanide adduct of ketone or aldehyde having 1 to 20 carbon atoms and the alkali metal cyanide are preferred and the alkali metal cyanide is more preferred.

The amount of the organic or inorganic cyano compound used is in the range of usually 0.1 to 10 moles, preferably 0.8 to 3 moles per mole of the 3-halogenated or 3-alkylsulfonated or 3-arylsulfonated tetrahydrofuran derivative.

In the method of cyanation of the compound of the formula (3) of the present invention, a compound accelerating a cyanation can be added to the reaction mixture. Examples of the compound accelerating a cyanation include a halogenated ammonium salt such as tetraethylammonium chloride, tetraethylammonium bromide, triethylammonium chloride and cetylpyridinium chloride, a halogenated phosphonium salt such as tetraphenylphosphonium chloride and tetraphenylphosphonium bromide, a cyclic ether compound such as 15-crown -5-ether and 18-crown -6-ether, a halogenated phosphazenium salt such as phosphazenium chloride, a halogenated alkali metal or alkaline earth metal such as sodium chloride, lithium chloride, potassium bromide, magnesium chloride, sodium iodide and potassium iodide, and amines such as 1,8-diazabicyclo[5.4.0]undec-7-ene and 1,4-diazabicyclo[2.2.2]octane. The amount of these compounds is in the range of usually 0.001 to 100 moles, preferably 0.01 to 50 moles per mole of the organic or inorganic cyano compound.

The method of cyanation of the compound represented by the formula (3) of the present invention may be carried out in the absence of a solvent, but is usually carried out in the presence of a solvent. Specific examples of the solvent used include monohydric or polyhydric alcohols having 1 to 20 carbon atoms such as methanol, ethanol, butanol and ethylene glycol, aliphatic or alicyclic hydrocarbons having 5 to 20 carbon atoms such as n-hexane, n-pentane and cyclohexane, aromatic hydrocarbons having 6 to 20 carbon atoms such as benzene, toluene and ethylbenzene, aliphatic or aromatic halides having 1 to 20 carbon atoms such as chloroform, chlorobenzene and dichlorobenzene, ethers having 2 to 20 carbon atoms such as diethyl ether, diphenyl ether, tetrahydrofuran and ethylene glycol dimethyl ether, aliphatic or aromatic amides having 2 to 20 carbon atoms such as N,N-dimethylformamide and N,N-dimethylacetamide, aliphatic or aromatic imidazolidinones having 2 to 20 carbon atoms such as 1,3-dimethyl-2-imidazolidinone, aliphatic or aromatic pyrrolidones having 4 to 20 carbon atoms such as N-methylpyrrolidone, aliphatic or aromatic esters having 2 to 20 carbon atoms such as ethyl acetate and butyl acetate, aliphatic or aromatic ketones having 3 to 20 carbon atoms such as acetone and methyl ethyl ketone, aliphatic or aromatic nitriles having 2 to 20 carbon atoms such as acetonitrile and benzonitrile, aliphatic or aromatic sulfoxides having 2 to 20 carbon atoms such as dimethylsulfoxide, and aliphatic or aromatic sulfones having 2 to 20 carbon atoms such as sulfolane, including water.

Of these solvents, it is preferable to use the solvent having permittivity of 20 F·m⁻¹ or more. The term "permittivity" as used in the present invention refers to permittivity at 20 to 30°C. The preferable solvent of the present invention does not have permittivity of 20 F·m⁻¹ or more over the total temperature range, and includes any solvent as long as it has permittivity of 20 F·m⁻¹ or more over a part of the temperature range. The permittivity of the solvent can take values described in "Solvent Handbook" (Shozo Asahara, et al., published by Kodansha Ltd. (1976)) or Handbook of Chemistry (Basic Edition), 5th Revised Edition (II) (edited by The Chemical Society of Japan, published by Maruzen Co., Ltd. (2004)). The permittivity is described as specific permittivity in some literatures, but both have the same meanings. Specific examples of the solvent having the permittivity of 20 F·m⁻¹ or more include methanol, ethanol, propanol, ethylene glycol, acetone, acetonitrile, N,N-dimethylformamide, N,N-dimethylacetamide, 1,3-dimethyl-2-imidazolidinone, dimethylsulfoxide and N-methylpyrrolidone, but is not limited thereto. Of solvents having the permittivity of 20 F·m⁻¹ or more solvent, it is more preferable to use an aprotic solvent. Further, of these solvents, it is most preferable to use aliphatic or aromatic amides, aliphatic or aromatic imidazolidinones, or aliphatic or aromatic sulfoxides, each of which has the permittivity of 20 F·m⁻¹ or more.

These solvents may be used in a mixture of two or more thereof. When the solvent is used, the amount thereof is not uniform over reaction conditions, but is in the range of usually 0.1 to 500 parts by weight, preferably 1 to 200 parts by weight, more preferably 2 to 100 parts by weight relative to 1 part by weight of the 3-halogenated or 3-alkylsulfonated or 3-arylsulfonated tetrahydrofuran derivative.

In the method of cyanation of the compound represented by the formula (3) of the present invention, it is particularly preferably to use the alkali metal cyanide as the cyano compound and react it in the presence of the solvent having the permittivity of 20 F·m⁻¹ or more. In this case, the solvent having the permittivity of 20 F·m⁻¹ or more is more preferably the aprotic solvent.

In the method of cyanation of the compound represented by the formula (3) of the present invention, all of R¹, R², R³, R⁴, R⁵, R⁶ and R⁷ are preferably a hydrogen atom. Further, X is preferably a halogen atom, more preferably a chlorine atom.

In the method of cyanation of the compound represented by the formula (3) of the present invention, the reaction temperature is in the range of usually 0°C to 250°C, preferably 20 to 200°C, more preferably 50 to 180°C. The reaction time is in the range of usually not more than 100 hours, preferably 0.01 to 50 hours. The pressure in the reaction may be any of reduced pressure, normal pressure or pressurization. The reaction process of the present invention is not particularly limited and may be performed in any one of batch, semibatch and continuous flow modes.

After completion of the reaction, the compound accelerating the reaction when used may be also recovered and repeatedly used in the following reaction.

The 3-cyanotetrahydrofuran derivative formed in the process of the present invention can be isolated according to the conventional separation method such as distillation and extraction.

In the present invention, it is preferable to produce the 3-cyanotetrahydrofuran derivative and subsequently produce the 3-aminomethyltetrahydrofuran derivative by using the 3-halogenated or 3-alkylsulfonated or 3-arylsulfonated tetrahydrofuran derivative represented by the formula (3a), which is prepared from the malic acid derivative by the following first to third steps, according to the process of the present invention.

### [First Step]

A -COOR⁸ group and a -COOR⁹ group of the malic acid derivative represented by the formula (4):

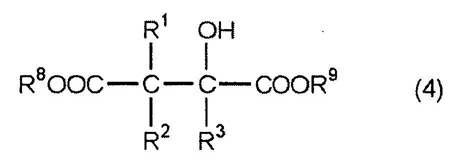

wherein R¹, R², R³, R⁸ and R⁹ may be the same or different from each other and each represent a hydrogen atom or a hydrocarbon group having 1 to 4 carbon atoms,
are reduced to prepare triols represented by the formula (5):

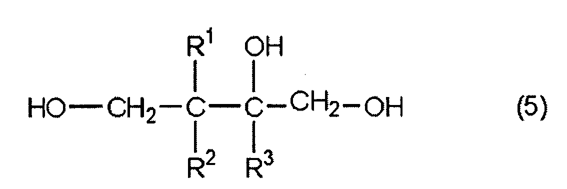

wherein R¹, R² and R³ may be the same or different from each other and each represent a hydrogen atom or a hydrocarbon group having 1 to 4 carbon atoms,
corresponding to the malic acid derivative used.

### [Second Step]

The triols represented by the formula (5) obtained from the first step is subjected to an intramolecular dehydration reaction in the presence of an acid catalyst to prepare a 3-hydroxytetrahydrofuran derivative represented by the formula (6):

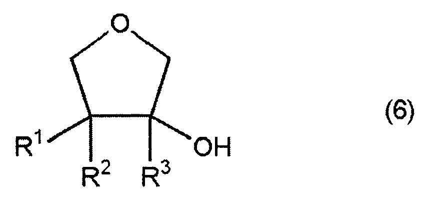

wherein R¹, R² and R³ may be the same or different from each other and each represent a hydrogen atom or a hydrocarbon group having 1 to 4 carbon atoms,
corresponding to the triols used.

### [Third Step]

The 3-hydroxytetrahydrofuran derivative represented by the formula (6) obtained from the second step and a halogenating agent or alkyl or arylsulfonylating agent are reacted to halogenate or alkyl or arylsulfonate a hydroxyl group thus to prepare the 3-halogenated or 3-alkylsulfonated or 3-arylsulfonated tetrahydrofuran derivative represented by the formula (3a):

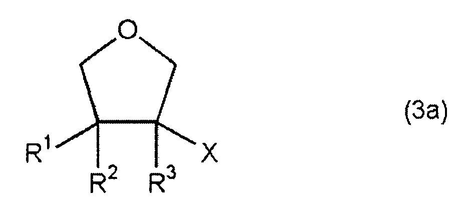

wherein R¹, R² and R³ may be the same or different from each other and each represent a hydrogen atom or a hydrocarbon group having 1 to 4 carbon atoms; and X represents a halogen atom, or an alkylsulfonate group having 1 to 6 carbon atoms or an arylsulfonate group having 6 to 12 carbon atoms.

Hereinafter, the process for producing the compound represented by the formula (3a) is explained in detail.

In the preferable process of the present invention, the malic acid derivative represented by the formula (4) is used as raw materials.

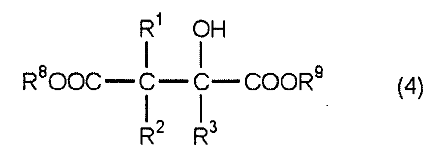

wherein R¹, R² and R³ are as defined for R¹, R² and R³ in the formula (1); R⁸ and R⁹ each represent a hydrogen atom or a hydrocarbon group having 1 to 4 carbon atoms.
More specific examples of the malic acid derivative include malic acid substituted by a hydrocarbon group at the 2-positon and/or 3-position, such as citramalic acid (2-methylmalic acid), 3-ethylmalic acid and 3, 3-dimethylmalic acid, malic monoester or diester such as monoisopropyl malate, dimethyl malate and diethyl malate, and monoester or diester substituted by a hydrocarbon group at the 2-positon and/or 3-position, such as dimethyl citramalate, monobutyl citramalate and dimethyl 3-ethylmalate, including malic acid. These malic acid derivatives have an asymmetric carbon, but the malic acid derivative used in the present invention may be an optically active substance or racemate. Of these malic acid derivatives, it is preferable to use malic acid, or malic monoester or diester, more preferably malic acid.

In the first step of the preferable process of the present invention, the -COOR⁸ group and -COOR⁹ group of the malic acid derivative represented by the formula (4) are reduced to prepare the triols represented by the formula (5):

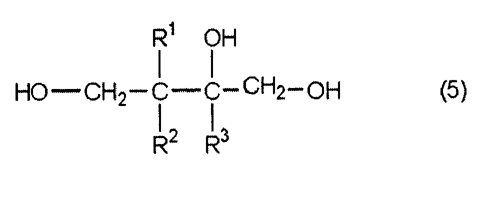

wherein R¹, R² and R³ are as defined for R¹, R² and R³ in the formula (1).
More specific examples of the triols include 1,2,4-butanetriol substituted by a hydrocarbon group at the 2-positon and/or 3-position, such as 2-methyl-1,2,4-butanetriol and 3-butyl-1,2,4-butanetriol, including 1,2,4-butanetriol. The triols obtained in the first step of the present invention corresponds to the malic acid derivative and a hydrogen atom or hydrocarbon group at the 2-position and the 3-position of the malic acid derivative used is the same as a hydrogen atom or hydrocarbon group of the 2-position and the 3-position of the triols obtained. For example, when malic monoester or diester, citramalic acid (2-methylmalic acid) and dimethyl 3-ethylmalate are used as the malic acid derivative, respectively, 1,2,4-butanetriol, 2-methyl-1,2,4-butanetriol and 3-ethyl-1,2,4-butanetriol are obtained, respectively.

In the first step of the preferable process of the present invention, examples of the method of reducing the -COOR⁸ group and -COOR⁹ group of the malic acid derivative represented by the formula (4) include a method of electrolytic reduction, a method of reducing with the metal hydride, a method of reducing with hydrogen in the presence of the hydrogenation catalyst.

Specific examples of the method of electrolytic reduction include a method of electrolytic reduction using a lead electrode in an aqueous sulfuric acid solution.

When reducing with the metal hydride, specific examples of the metal hydride include the aluminum hydride compound such as lithium aluminum hydride, lithium trimethoxyaluminum hydride, aluminum hydride and diisobutylaluminum hydride and the boron hydride compound such as diborane, lithium borohydride and sodium borohydride. The amount of the metal hydride when reducing with the metal hydride is in the range of usually 2 to 10 moles, preferably 3 to 6 moles per mole of the malic acid derivative.

The hydrogenation catalyst when reducing with hydrogen in the presence of the hydrogenation catalyst may be also any compound as long as it catalyzes the reaction in which the carboxyl group or ester group of the malic acid derivative according to the present invention is reduced to a hydroxyl group by a molecular hydrogen, but usually at least one metal selected from Groups 7 to 13 of the Periodic Table of the Elements or a metal compound thereof is suitably used. Specific examples of the hydrogenation catalyst and the amount used thereof may be exemplified by the same as those exemplified in the method of reducing the cyano groups of the 3-cyanotetrahydrofuran derivative represented by the formula (1).

Of these reduction methods, the method of reducing with hydrogen in the presence of the hydrogenation catalyst is preferred. The hydrogenation catalyst is preferably a metal such as ruthenium, rhodium, palladium, copper or rhenium or a metal compound thereof, more preferably a metal such as ruthenium or rhodium or metal compound thereof, and most preferably a metal such as ruthenium or metal compound thereof.

The first step of the preferable process of the present invention may be carried out without a solvent, but is usually carried out in the presence of the solvent. When the solvent is used, the solvent may be any solvent as long as it does not inhibit the reduction reaction.

As the solvent used in the method of electrolytic reduction, water and alcohols having 1 to 20 carbon atoms, methanol, ethanol and butanol, are suitably used. Further, these solvents may be used in a mixture of two or more thereof.

When reducing with the metal hydride, aliphatic or alicyclic hydrocarbons having 5 to 20 carbon atoms such as n-hexane, n-pentane and cyclohexane, aromatic hydrocarbons having 6 to 20 carbon atoms such as benzene, toluene and ethylbenzene, aliphatic or aromatic halide havings 1 to 20 carbon atoms such as chloroform, chlorobenzene and dichlorobenzene, and ethers having 2 to 20 carbon atoms such as diethyl ether, diphenyl ether, tetrahydrofuran and ethylene glycol dimethyl ether are suitably used. Further, these solvents may be used in a mixture of two or more thereof. Of these solvents, it is preferable to use ethers.

As the solvent used when reducing with hydrogen in the presence of the hydrogenation catalyst solvent, water, alcohols having 1 to 20 carbon atoms such as methanol, ethanol and butanol, aliphatic or alicyclic hydrocarbons having 5 to 20 carbon atoms such as n-hexane, n-pentane and cyclohexane, aromatic hydrocarbons having 6 to 20 carbon atoms such as benzene, toluene and ethylbenzene and ethers having 2 to 20 carbon atoms such as diethyl ether, diphenyl ether, tetrahydrofuran and ethylene glycol dimethyl ether are suitably used. Further, these solvents may be used in a mixture of two or more thereof. Of these solvents, it is preferable to use water, alcohols and ethers, more preferably water or alcohols having 1 to 4 carbon atoms.

When the solvent is used, the amount thereof is not uniform over reaction conditions, but is in the range of usually 0.1 to 500 parts by weight, preferably 1 to 200 parts by weight relative to 1 part by weight of the malic acid derivative.

The temperature and time in the reaction are not uniform over the kinds of the malic acid derivative used and the reduction method. However, the reaction temperature is in the range of usually -10 to 250°C, preferably 10 to 200°C. The reaction time is in the range of usually not more than 150 hours, preferably 0.01 to 100 hours.

The pressure in the reaction may be any of reduced pressure, normal pressure or pressurization, but the preferable mode varies depending on the reduction method to be performed. When reducing with the metal hydride, it is preferably carried out at normal pressure. When electrolytic reduction or reducing with the metal hydride, it is preferably carried out at normal pressure. When reducing with hydrogen in the presence of the hydrogenation catalyst, it is preferably carried out under hydrogen pressurization. The hydrogen pressure in pressurization is in the range of usually 0.01 to 30 MPaG, preferably 0.1 to 25 MPaG.

The reaction process of the first step of the preferable process of the present invention is not particularly limited and may be performed in any one of batch, semibatch and continuous flow modes.

When reducing with hydrogen in the presence of the hydrogenation catalyst in the first step of the preferable process of the present invention, the catalyst used may be recovered by the conventional process for recovery of metals after completion of the reaction. For example, when the catalyst is dissolved in the reaction liquid, the catalyst can be recovered by contacting with a metal adsorbent such as an ion-exchange resin or by extracting from the solvent. When the catalyst is not dissolved in the reaction liquid, the catalyst can be recovered by a solid-liquid separation method such as filtration and centrifugal separation. These recovered catalysts can be repeatedly used as the hydrogenation catalyst of the first step of the present invention. In that case, it may be also reused after the catalyst deactivated or having reduced activity is subjected to a recycling operation. The reaction liquid itself may be provided in the following step without recovering the catalyst as long as the remaining catalyst does not affect the subsequent recovery step of the triols and/or the second step of the preferable process of the present invention.

The triols formed in the first step of the preferable process of the present invention may be provided in the following second step after isolation according to the conventional separation method such as distillation, may be provided in the following step as a mixture after distilling off only the solvent when the solvent is used, or may be provided in the following step as the reaction mixture itself, which contains the solvent.

In the second step of the preferable process of the present invention, the triols obtained from the first step is subjected to an intramolecular dehydration reaction in the presence of an acid catalyst to prepare the 3-hydroxytetrahydrofuran derivative represented by the formula (6):

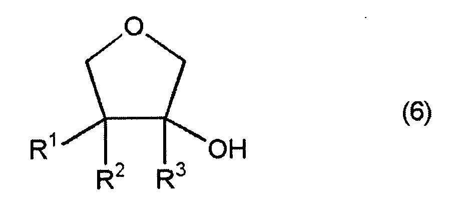

wherein R¹, R² and R³ are as defined for R¹, R² and R³ in the formula (1).
Examples of the 3-hydroxytetrahydrofuran derivative include a 3-hydroxytetrahydrofuran substituted by a hydrocarbon group at the 3-positon and/or 4-position, such as 4-ethyl-3-hydroxytetrahydrofuran and 3-methyl-3-hydroxytetrahydrofuran, including 3-hydroxytetrahydrofuran. The 3-hydroxytetrahydrofuran derivative obtained in the second step corresponds to the triols used and a hydrogen atom or hydrocarbon group at the 2-position and the 3-position of the triols used is the same as a hydrogen atom or hydrocarbon group of the 3-position and the 4-position of the 3-hydroxytetrahydrofuran derivative obtained. For example, when 1,2,4-butanetriol and 2-methyl-1,2,4-butanetriol are used as the triols, respectively, 3-hydroxytetrahydrofuran and 3-methyl-3-hydroxytetrahydrofuran are obtained, respectively.

The acid catalyst in the second step of the preferable process of the present invention may be any one of Bronsted acid and Lewis acid and may be soluble or insoluble in the reaction mixture. The acid catalyst may be any one of an organic acid, an inorganic acid and a solid acid and specific examples thereof include inorganic acids such as hydrochloric acid , hydrobromic acid, boric acid , nitric acid , sulfuric acid, phosphoric acid and chloric acid, organic carboxylic acids such as formic acid, acetic acid, propionic acid, chloroacetic acid, glycolic acid, benzoic acid and phthalic acid, organic sulfonic acids such as methylsulfonic acid, benzenesulfonic acid, p-toluenesulfonic acid and trifluoromethanesulfonic acid, organic phosphoric acids such as diethyl phosphate and phenyl phosphate, metal halides such as aluminum chloride, zinc iodide and titanium chloride, metal salts of trichloromethanesulfonic acid such as yttrium trifluoromethanesulfonate and samarium trifluoromethanesulfonate, acidic ion-exchange resin such as a sulfonic acid type ion-exchange resin, oxide complexes composed of two or more metal oxides, such as silica-magnesia, silica-alumina, silica-titania, titania-zirconia and alumina-boria, natural clay minerals such as acid clay and montmorillonite and kaolin, a support acid having phosphoric acid or sulfuric acid supported on a support such as diatom earth, silica gel, sellite, alumina and zirconia, acidic natural or synthetic zeolite such as Y zeolite, mordenite and ZSM-5, a single metal oxide such as niobic acid, zinc oxide, alumina and titanium oxide, and solid superacid having antimony pentafluoride and boron trifluoride supported on a support such as silica-alumina, silica-zirconia and graphite. The amount of the acid catalyst used is in the range of usually 0.0001 to 10 mol%, preferably 0.001 to 5 mol% when soluble in the triols as raw materials of the second step and is in the range of usually 0.001 to 50% by weight, preferably 0.1 to 20% by weight when insoluble in the triols.

The second step of the preferable process of the present invention can be carried out in the presence of the solvent. When the solvent is used, the solvent may be any solvent as long as it does not inhibit the intramolecular dehydration reaction of the triols. As specific examples of the solvent, among the solvents exemplified as those usable in the first step, aliphatic or alicyclic hydrocarbons having 5 to 20 carbon atoms, aromatic hydrocarbons having 6 to 20 carbon atoms, aliphatic or aromatic halides having 1 to 20 carbon atoms and ethers having 2 to 20 carbon atoms, are suitably used. Further, these solvents may be used in a mixture of two or more thereof. The amount of the solvent used is not uniform over reaction conditions, but is in the range of usually 0.1 to 500 parts by weight, preferably 1 to 200 parts by weight per part by weight of the triols.

The second step of the preferable process of the present invention is preferably carried out while water formed by the intramolecular dehydration reaction is removed out of the reaction system. A method of removing the water formed out of the reaction system is not particularly limited, but examples thereof include a method of distilling off water formed by carrying out the reaction under reduced pressure out of the reaction system, a method of removing an azeotropic mixture obtained by carrying out the reaction in the presence of the solvent such as toluene which is azeotropic with water, out of the reaction system and a method of coexisting a dehydrating agent in the reaction liquid.

The reaction temperature in the second step of the preferable process of the present invention is in the range of usually 10 to 250°C, preferably 30 to 200°C. The reaction time is in the range of usually not more than 100 hours, preferably 0.01 to 50 hours.

The pressure in the reaction may be any of reduced pressure, normal pressure or pressurization, but the preferable mode varies depending on the reduction method to be performed. The reaction is preferably carried out under reduced pressure when it is carried out while distilling off the water formed.

The reaction process of the present invention is not particularly limited and may be performed in any one of batch, semibatch and continuous flow modes.

After completion of the reaction, the catalyst used may be also recovered and repeatedly used in the following reaction. For example, the catalyst may be neutralized with a base such as sodium hydroxide. When the catalyst is the solid acid, the catalyst may be separated by a conventional solid-liquid separation or the catalyst itself may be used in the following purification step without neutralization or separation.

The 3-hydroxytetrahydrofuran derivative formed in the second step of the preferable process of the present invention may be provided in the following third step after isolation according to a conventional separation such as distillation or may be further provided in the following step as the reaction mixture.

In the third step of the preferable process of the present invention, the 3-hydroxytetrahydrofuran derivative obtained from the second step and a halogenating agent or alkyl or arylsulfonylating agent are reacted to halogenate or alkyl or arylsulfonate a hydroxyl group thus to prepare the 3-halogenated or 3-alkylsulfonated or 3-arylsulfonated tetrahydrofuran derivative represented by the formula (3a):

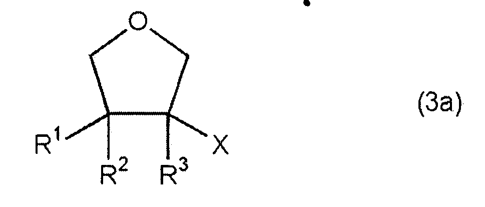

wherein R¹, R², R³ and X are as defined for R¹, R², R³ and X in the formula (3).
The 3-halogenated or 3-alkylsulfonated or 3-arylsulfonated tetrahydrofuran derivative represented by the formula (3a) is a compound wherein all of R⁴, R⁵, R⁶ and R⁷ are a hydrogen atom, among the compounds represented by the formula (3). The 3-halogenated or 3-alkylsulfonated or 3-arylsulfonated tetrahydrofuran derivative obtained in the third step of the preferable process of the present invention corresponds to the 3-hydroxytetrahydrofuran derivative used and a hydrogen atom or hydrocarbon group at the 3-position and the 4-position of the 3-hydroxytetrahydrofuran derivative used is the same as a hydrogen atom or hydrocarbon group of the 3-position and the 4-position of the 3-halogenated or 3-alkylsulfonated or 3-arylsulfonated tetrahydrofuran derivative obtained. For example, when 3-hydroxytetrahydrofuran and 3-methyl-3-hydroxytetrahydrofuran are used as the 3-hydroxytetrahydrofuran derivative, respectively, 3-halogenated or 3-alkylsulfonated or 3-arylsulfonated tetrahydrofuran and 3-halogenated or 3-alkylsulfonated or 3-arylsulfonated-3-methyltetrahydrofuran are obtained, respectively.

The halogenating agent in the third step of the preferable process of the present invention may be any compound as long as the hydroxyl group of the 3-hydroxytetrahydrofuran derivative can be efficiently converted to a halogen group and examples of the halogenating agent include the halogenating agent used in the synthesis of a halogen compound from an alcohol compound, which is described in Experimental Chemistry Course, Vol. 20 (edited by The Chemical Society of Japan, published by Maruzen Co., Ltd. (1956)) or New Experimental Chemistry Course, Vol. 14 (I) (edited by The Chemical Society of Japan, published by Maruzen Co., Ltd. (1977)). More specific examples of the halogenating agent include a fluorinating agent such as an EiF/pyridine solution, 1,1,2,2-tetrafluoroethyldiethylamine and trifluorodiphenylphosphorane, a chlorinating agent such as phosgene, thionyl chloride, zinc chloride/hydrochloric acid , t-butyl hypochlorite and N-chlorinated amine, a brominating agent such as hydrobromic acid, t-butyl hypobromite and thionyl bromide and an iodinating agent such as hydriodic acid. Of these halogenating agents, it is preferable to use the chlorinating agent. Of the chlorinating agents, it is preferable to use phosgene or thionyl chloride. The amount of the halogenating agent used is in the range of usually 0.1 to 10 moles, preferably 0.8 to 3 moles per mole of the 3-hydroxytetrahydrofuran derivative.

The alkyl or arylsulfonylating agent in the third step of the preferable process of the present invention may be any compound as long as it can efficiently convert the hydroxyl group of the 3-hydroxytetrahydrofuran derivative into an alkyl or arylsulfonate group. More specific examples of the alkyl or arylsulfonylating agent include alkylsulfonic acids having 1 to 6 carbon atoms or arylsulfonic acids having 6 to 12 carbon atoms, such as p-toluenesulfonic acid, benzenesulfonic acid, methanesulfonic acid, ethanesulfonic acid and trifluoromethanesulfonic acid, halides of the alkyl or arylsulfonic acids, such as tosyl chloride (p-toluenesulfonic acid chloride), ammonium salts of the alkyl or arylsulfonic acids such as ammonium trifluoromethanesulfonate and tetraethylammonium benzenesulfonate, alkali metal salts of the alkyl or arylsulfonic acids such as sodium p-toluenesulfonate, anhydrides of the alkyl or arylsulfonic acids such as p-toluenesulfonic anhydride, and ester compounds of the alkyl or arylsulfonic acids such as ethyl methanesulfonate. Of these alkyl or arylsulfonylating agents, it is preferable to use halides and anhydrides of the alkyl or arylsulfonic acids, more preferably halides of the alkyl or arylsulfonic acids. The amount of the alkyl or arylsulfonylating agent used is in the range of usually 0.1 to 10 moles, preferably 0.8 to 3 moles per mole of the 3-hydroxytetrahydrofuran derivative.

In the process of the present invention in which the 3-hydroxytetrahydrofuran derivative and a halogenating agent or alkyl or arylsulfonylating agent are reacted to halogenate or alkyl or arylsulfonate a hydroxyl group thus to prepare the 3-halogenated or 3-alkylsulfonated or 3-arylsulfonated tetrahydrofuran derivative represented by the formula (3a), a method of halogenation by the halogenating agent is preferred and a method of chlorination by the chlorinating agent is more preferred.

In the third step of the preferable process of the present invention, the suitable catalyst and reaction accelerator can be used in order to efficiently react the 3-hydroxytetrahydrofuran derivative and the halogenating agent or alkyl or arylsulfonylating agent. The catalyst and reaction accelerator when used varies depending on the halogenating agent or alkyl or arylsulfonylating agent. For example, when phosgene or thionyl chloride are used as the halogenating agent, it is preferred in that amines such as pyridine and triethylamine and amides such as N,N-dimethylformamide and N,N-dimethylacetamide are used to accelerate the reaction and thus improve the yield. Further, when halides of the alkyl or arylsulfonic acids such as tosyl chloride are used, it is preferable to use amines such as pyridine and triethylamine.

The third step of the preferable process of the present invention may be carried out in the absence or in the presence of a solvent. When the solvent is used, the solvent may be any solvent as long as it does not inhibit the reaction of the 3-hydroxytetrahydrofuran derivative and the halogenating agent or alkyl or arylsulfonylating agent. As specific examples of the solvent, among the solvents exemplified as those usable in the first step, aliphatic or aromatic amides having 2 to 20 carbon atoms such as N,N-dimethylformamide and N,N-dimethylacetamide, aliphatic or aromatic esters having 2 to 20 carbon atoms such as ethyl acetate and butyl acetate, aliphatic or aromatic nitriles having 2 to 20 carbon atoms such as acetonitrile and benzonitrile and aliphatic or aromatic sulfoxides having 2 to 20 carbon atoms such as dimethylsulfoxide, in addition to aliphatic or alicyclic hydrocarbons having 5 to 20 carbon atoms, aromatic hydrocarbons having 6 to 20 carbon atoms, aliphatic or aromatic halides having 1 to 20 carbon atoms and ethers having 2 to 20 carbon atoms, are suitably used. Further, these solvents may be used in a mixture of two or more thereof. The amount of the solvent used is not uniform over reaction conditions, but is in the range of usually 0.1 to 500 parts by weight, preferably 1 to 200 parts by weight per part by weight of the 3-hydroxytetrahydrofuran derivative.

In the third step of the preferable process of the present invention, the reaction temperature is in the range of usually -80 to 150°C, preferably -20 to 80°C. The reaction time is in the range of usually not more than 200 hours, preferably 0.01 to 100 hours. The pressure in the reaction may be any of reduced pressure, normal pressure or pressurization. The reaction process of the present invention is not particularly limited and may be performed in any one of batch, semibatch and continuous flow modes.

After completion of the reaction, the catalyst or reaction accelerator when used may be also recovered and repeatedly used in the following reaction, and may be separated by the conventional separation method.

The 3-halogenated or 3-alkylsulfonated or 3-arylsulfonated tetrahydrofuran derivative formed in the third step of the preferable process of the present invention may be provided in the following step after isolation according to the conventional separation method such as distillation and extraction.

### [Examples]

Hereinafter, the present invention will be explained in more detail by way of Examples.

### [Preparative Example of 3-aminomethyltetrahydrofuran derivative]

### [Example 1]

20.4 g (210 mmol) of 3-cyanotetrahydrofuran, 14.3 g (210 mmol in terms of NH₃) of 25% aqueous ammonia and 1.0 g of Raney nickel were charged into an autoclave. After purging with nitrogen, hydrogen pressure was adjusted to 5 MPaG and the reaction mixture was reacted at 100°C for 3 hours. The catalyst was filtered off after reaction and the filtrate was analyzed by gas chromatography. The conversion rate of raw materials was 100% and the yield of the 3-aminomethyltetrahydrofuran as the target product was 89.8%. Further, an amide compound of the following structure wherein the cyano group of 3-cyanotetrahydrofuran as a major by-product was hydrated, was formed in the yield of 0.7%. The 3-aminomethyltetrahydrofuran formed was distilled under reduced pressure to isolate as a transparent liquid being a fraction of 67°C/2.0 kPa.

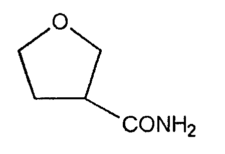

### [Examples 2 to 13]

Reaction and after-treatment were carried out in the same manner as in Example 1, except that, in Example 1 wherein an equal amount of 3-cyanotetrahydrofuran was used, the type and the amount of the catalyst, the amount of 25% aqueous ammonia, the hydrogen pressure, the reaction temperature and the reaction time were replaced as shown in Table 1. The results are shown in Table 1 together with those of Example 1.

**[Table 1]**

| | Catalyst^{(*)} | | 25% NH₃ water (g) | Pressure (MPa) | Temperature (°C) | Time (h) | Conversion rate of raw materials (%) | Yield (%) | Yield of amide compound (%) |
|---|---|---|---|---|---|---|---|---|---|
| | Type | Amount (g) | | | | | | | |
| Ex. 1 | R-Ni | 1.0 | 14.3 | 5 | 100 | 3 | 100.0 | 89.8 | 0.7 |
| Ex. 2 | R-Ni | 1.0 | 70.0 | 5 | 100 | 3 | 88.9 | 70.9 | 4.5 |
| Ex. 3 | R-Ni | 1.5 | 70.0 | 5 | 50 | 7 | 100.0 | 54.9 | 39.4 |
| Ex. 4 | R-Ni | 1.5 | 70.0 | 5 | 90 | 4 | 100.0 | 66.7 | 17.1 |
| Ex. 5 | R-Ni | 1.5 | 70.0 | 10 | 120 | 1 | 100.0 | 82.4 | 7.9 |
| Ex. 6 | R-Co | 1.0 | 14.3 | 5 | 90 | 3 | 77.2 | 70.4 | 1.3 |
| Ex. 7 | R-Co | 1.0 | 14.3 | 5 | 100 | 3 | 99.4 | 95.2 | 2.0 |
| Ex. 8 | R-Co | 1.0 | 14.3 | 5 | 110 | 2 | 99.4 | 93.4 | 2.0 |
| Ex. 9 | R-Co | 1.5 | 70.0 | 4 | 50 | 4 | 28.7 | 21.0 | 3.7 |
| Ex. 10 | R-Co | 1.0 | 42.9 | 5 | 100 | 3 | 100.0 | 95.6 | 2.8 |
| Ex. 11 | R-Co | 1.0 | 4.3 | 5 | 100 | 3 | 99.5 | 91.4 | 1.4 |
| Ex. 12 | R-Co | 1.0 | 14.3 | 3 | 100 | 3 | 92.4 | 79.7 | 2.1 |
| Ex. 13 | R-Co | 1.0 | 14.3 | 10 | 100 | 3 | 99.4 | 93.8 | 1.4 |

| | | | | | | | | | |
|---|---|---|---|---|---|---|---|---|---|
| (*) R-Ni: Raney nickel, R-Co: Raney cobalt | | | | | | | | | |

### [Example 14]

Reaction and after-treatment were carried out in the same manner as in Example 1, except that 14.3 g of water was used instead of aqueous ammonia. The yield of 3-aminomethyltetrahydrofuran was 13.6%. As a by-product, 68.0% of secondary amine of the following structure, in addition to 4.8% of an amide compound, was formed.

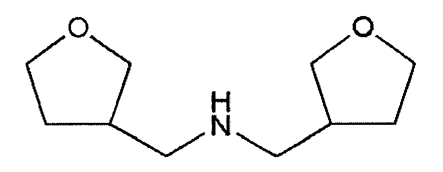

### [Example 15]

Reaction and after-treatment were carried out in the same manner as in Example 1, except that aqueous ammonia was not used. The yield of 3-aminomethyltetrahydrofuran was 11.1%. As a by-product, 78.5% of secondary amine was formed.

[Preparative Example of 3-cyanotetrahydrofuran derivative]

### [Example 16]

26. 6 g (250 mmol) of 3-chlorotetrahydrofuran, 18.4 g (375 mmol) of NaCN and 260 mL of N,N-dimethylformamide (hereinafter abbreviated to "DMF") were charged into an autoclave. The reaction mixture was heated to 150°C and reacted for 5 hours. A salt precipitated after completion of the reaction was filtered off and the filtrate was analyzed by gas chromatography. The yield of the 3-cyanotetrahydrofuran was 87.1%. Further, 10.1% of 2,5-dihydrofuran as a major by-product was formed. 21.1 g (217 mmol) of 3-cyanotetrahydrofuran formed was distilled under reduced pressure to isolate as a transparent liquid being a fraction of 123°C/10.8 kPa.

### [Examples 17 to 22]

Reaction and after-treatment were carried out in the same manner as in Example 16, except that the type of the solvent, the reaction temperature and the reaction time were replaced as shown in Table 2. The results are shown in Table 2 together with those of Example 16. Further, permittivity of each solvent was also shown in Table 2.

**[Table 2]**

| | Solvent | Permittivity^{(*)} (F·m⁻¹) | Temperature (°C) | Time (h) | Conversion rate (%) | Yield (%) | 2,5-DHF^{(#)} yield (%) |
|---|---|---|---|---|---|---|---|
| Ex. 16 | N,N-dimethylformamide | 36.7 (25) | 150 | 5 | 100 | 87.1 | 10.1 |
| Ex. 17 | Dimethylsulfoxide | 48.9 (20) | 130 | 4 | 100 | 89.6 | 8.8 |
| Ex. 18 | 1,3-dimethyl-2-imidazolidin one | 36.7 (25) | 120 | 4 | 76.7 | 59.6 | 12.7 |
| Ex. 19 | N-methylpyrrolidone | 32.0 (25) | 120 | 4 | 71.9 | 54.3 | 11.6 |
| Ex. 20 | Methanol | 23.8 (25) | 140 | 7 | 58.6 | 22.8 | 15.3 |
| Ex. 21 | Sulfolane | 43.3 (30) | 140 | 7 | 86.3 | 55.6 | 10.3 |
| Ex. 22 | N,N-dimethylacetamide | 37.8 (25) | 130 | 3 | 72.7 | 52.7 | 8.4 |
| Ex. 23 | No solvent | - | 150 | 5 | 6.4 | 4.0 | 0.1 |

| | | | | | | | |
|---|---|---|---|---|---|---|---|
| (*) The figures in parentheses indicate the measured temperature of permittivity. (#) 2,5-DHF: 2,5-dihydrofuran | | | | | | | |

### [Example 23]

Reaction and after-treatment were carried out in the same manner as in Example 16, except that the solvent was not used. The results are shown in Table 2.

### [Example 24]

Reaction and after-treatment were carried out in the same manner as in Example 16, except that, in Example 16 wherein DMF was used as a solvent, the reaction temperature was adjusted to 170°C and the reaction time was adjusted to 4 hours. The conversion rate was 100% and the yield of 3-cyanotetrahydrofuran was 74.6%.

### [Example 25]

Reaction and after-treatment were carried out in the same manner as in Example 16, except that, in Example 16 wherein DMF was used as a solvent, the amount of the solvent was adjusted to 130 mL. The conversion rate was 100% and the yield of 3-cyanotetrahydrofuran was 87.0%.

### [Example 26]

Reaction and after-treatment were carried out in the same manner as in Example 25, except that 24.4 g (375 mmol) of KCN was used instead of NaCN. The conversion rate was 80.8% and the yield of 3-cyanotetrahydrofuran was 54.8%.

### [Example 27]

Reaction and after-treatment were carried out in the same manner as in Example 25, except that 31.9 g (375 mmol) of acetonecyanhydrin was used instead of NaCN and 41.9 g (275 mmol) of 1,8-diazabicyclo[5.4.0]undec-7-ene was used. The conversion rate was 81.7% and the yield of 3-cyanotetrahydrofuran was 54.5%.

### [Example 28]

Reaction and after-treatment were carried out in the same manner as in Example 17, except that, in Example 17 wherein dimethylsulfoxide (hereinafter abbreviated to "DMSO") was used as a solvent, the reaction temperature was adjusted to 120°C. The conversion rate was 98.1% and the yield of 3-cyanotetrahydrofuran was 89.3%.

### [Example29]

Reaction and after-treatment were carried out in the same manner as in Example 17, except that, in Example 17 wherein DMSO was used as a solvent, the reaction temperature was adjusted to 110°C and the reaction time was adjusted to 8 hours. The conversion rate was 94.3% and the yield of 3-cyanotetrahydrofuran was 86.3%.

### [Example 30]

Reaction and after-treatment were carried out in the same manner as in Example 17, except that, in Example 17 wherein DMSO was used as a solvent, the amount used of NaCN was adjusted to 13.5 g (275 mmol). The conversion rate was 99.7% and the yield of 3-cyanotetrahydrofuran was 87.0%.

### [Example 31]

Reaction and after-treatment were carried out in the same manner as in Example 25, except that 60.6 g (250 mmol) of 3-(p-toluenesulfonato)-tetrahydrofuran was used instead of 3-chlorotetrahydrofuran. The yield of 3-cyanotetrahydrofuran was 71.3%.

### [Example 32]

Reaction and after-treatment were carried out in the same manner as in Example 25, except that 55.0 g (250 mmol) of 3-(trifluoromethanesulfonato)-tetrahydrofuran was used instead of 3-chlorotetrahydrofuran. The yield of 3-cyanotetrahydrofuran was 68.0%.

[Preparative Example of 3-halogenated or 3-alkylsulfonated or 3-arylsulfonated tetrahydrofuran derivative from malic acid derivative]

### [Example 33]

### [First Step (step of reducing malic acid)]

56.0 g (418 mmol) of malic acid, 400 mL of water and 40 g of 5% ruthenium-carbon powders were charged into a 1L autoclave. After purging the inside of a reactor with nitrogen, the inside of the reaction system was pressurized with hydrogen of 1 MPaG. The reaction solution was heated to 100°C and then reacted at hydrogen pressure of 12 MPaG for 24 hours. After completion of the reaction, the catalyst was filtered off and the filtrate was distilled under reduced pressure to obtain 34.2 g (322 mmol) of 1,2,4-butanetriol as a fraction of 185 to 190°C/2.4 kPa. The yield was 77%.

### [Second Step (step of cyclizing triol)]

31.8 g (300 mmol) of 1,2,4-butanetriol obtained in the first step and 0.3 g of p-toluenesulfonic acid monohydrate were placed into a 75 mL three-necked flask equipped with a cooling extraction pipe. The inside of the reaction system was adjusted to a reduced pressure of 6.6 kPa and the inside of the reactor was heated to 140°C, and then the cyclization reaction of 1,2,4-butanetriol was carried out. The reaction was carried out in the form of reactive distillation and 3-hydroxytetrahydrofuran as a cyclization product was extracted through the cooling extraction pipe out of the reaction system, together with the formed water. The moisture was removed from the moisture-containing 3-hydroxytetrahydrofuran obtained by means of distillation to obtain 25.6 g (291 mmol) of 3-hydroxytetrahydrofuran. The yield was 97%.

### [Third Step (step of halogenating 3-hydroxytetrahydrofuran)]

24.7 g (280 mmol) of 3-hydroxytetrahydrofuran obtained in the second step and 85 mL of DMF were placed into a 200 mL three-necked flask. After immersing the flask in an ice bath, 36.7 g (308 mmol) of thionyl chloride was added dropwise over 30 minutes to the N,N-dimethylformamide solution. After completion of dropwise addition, the reaction was continued for further 6 hours. After completion of the reaction, the sulfur dioxide and hydrogen chloride formed were ejected by nitrogen bubbling and then the resulting product was distilled under normal pressure to obtain 27.1 g (254 mmol) of 3-chlorotetrahydrofuran as a fraction of 125 to 130°C. The yield was 91%.

Hereinafter, an example of each step of preparing the 3-halogenated or 3-alkylsulfonated or 3-arylsulfonated tetrahydrofuran derivative from the malic acid derivative will be separately and specifically explained. The 3-halogenated or 3-alkylsulfonated or 3-arylsulfonated tetrahydrofuran derivative can be synthesized from the malic acid derivative by any suitable combination of each step of Example 33, and Example 34 and the subsequent Examples.

### [Example 34]

### [First Step]

Reaction and distillation were carried out in the same manner as in the first step of Example 33, except that, in the first step of Example 33, 40 g of 5% rhodium-carbon powders was used instead of 40 g of 5% ruthenium-carbon powders and hydrogen pressure was adjusted to 16 MPaG. The yield of 1,2,4-butanetriol was 56%.

### [Example 35]

### [First Step]

Reaction and distillation were carried out in the same manner as in the first step of Example 33, except that, in the first step of Example 33, the reaction temperature was changed to 120°C and the reaction time was changed to 6 hours. The yield of 1,2,4-butanetriol was 63%.

### [Example 36]

### [First Step]

Reaction and distillation were carried out in the same manner as in the first step of Example 33, except that, in the first step of Example 33, 400 mL of ethanol was used instead of 400 mL of water as a solvent, hydrogen pressure was changed to 16 MPaG and the reaction time was changed to 90 hours. The yield of 1,2,4-butanetriol was 90%.

### [Example 37]

### [First Step]

Reaction and distillation were carried out in the same manner as in the first step of Example 33, except that, in the first step of Example 33, 400 mL of ethanol was used instead of 400 mL of water as a solvent, hydrogen pressure was changed to 16 MPaG, the reaction temperature was changed to 120°C and the reaction time was changed to 70 hours. The yield of 1,2,4-butanetriol was 90%.

### [Example 38]

### [First Step]

50.0 g (308 mmol) of dimethyl malate, 200 mL of tetrahydrofuran and 25 g of CuO/ZnO/Al₂O₃ (CuO/ZnO/Al₂O₃ were 52.8% by weight, 28.1% by weight and 19.1% by weight, respectively) were charged into a 500 mL autoclave. After purging the inside of a reactor with nitrogen, the inside of the reaction system was pressurized with hydrogen of 1 MPaG. The reaction solution was heated to 180°C and then reacted at hydrogen pressure of 10 MPa for 4 hours. After completion of the reaction, the catalyst was filtered off and the filtrate was distilled under reduced pressure. The yield of 1,2,4-butanetriol was 80%.

### [Example 39]

### [Second Step]

Reaction and distillation were carried out completely in the same manner as in the second step of Example 33, except that, in the second step of Example 33, 0.3 g of sulfuric acid was used instead of p-toluenesulfonic acid monohydrate. The yield of 3-hydroxytetrahydrofuran was 98%.

### [Example 40]

### [Second Step]

Reaction and distillation were carried out in the same manner as in the second step of Example 33, except that, in the second step of Example 33, 0.5 g of samarium trifluoromethanesulfonate was used instead of p-toluenesulfonic acid monohydrate. The yield of 3-hydroxytetrahydrofuran was 97%.

### [Example 41]

### [Second Step]

Reaction and distillation were carried out completely in the same manner as in the second step of Example 33, except that, in the second step of Example 33, 1.0 g of H-type mordenite was used instead of p-toluenesulfonic acid monohydrate. The yield of 3-hydroxytetrahydrofuran was 95%.

### [Example 42]

### [Second Step]

Reaction and distillation were carried out completely in the same manner as in the second step of Example 33, except that, in the second step of Example 33, the pressure during the reaction was changed to 13.2 kPa. The yield of 3-hydroxytetrahydrofuran was 95%.

### [Example 43]

### [Third Step]

Reaction was carried out in the same manner as in the third step of Example 33, except that, in the third step of Example 33, 22.1 g (280 mmol) of pyridine and 50 mL of toluene were used instead of DMF. After completion of the reaction, the sulfur dioxide and hydrogen chloride formed were ejected by nitrogen bubbling, the precipitated pyridinium salt was filtered off, and then the resulting product was distilled under normal pressure to obtain 3-chlorotetrahydrofuran. The yield was 93%.

### [Example 44]

### [Third Step]

50 mL of concentrated hydrochloric acid and 76.4 g (560 mmol) of zinc chloride were mixed in a 250 mL three-necked flask and then 24.7 g (280 mmol) of 3-hydroxytetrahydrofuran was added dropwise to the resulting solution. The solution was refluxed for 2 hours, and the upper layer was separated and heated under reflux with concentrated hydrochloric acid. The product was distilled to obtain 3-chlorotetrahydrofuran. The yield was 78%.

### [Example 45]

### [Third Step]

24.7 g (280 mmol) of 3-hydroxytetrahydrofuran and 53.5 g (280 mmol) of tosyl chloride were dissolved in 150 mL of ether and a solution of 44.3 g (560 mmol) of pyridine dissolved in 50 mL of ether was added dropwise at 0°C to the resulting solution. After completion of the reaction, pyridine hydrochloride was separated by filtration, the solvent was distilled off and the residue was recrystallized with ether to obtain 40.0 g (165 mmol) of 3-(p-toluenesulfonato)-tetrahydrofuran. The yield was 59%.

### [Example 46]

### [Third Step]

3.6 g (150 mmol) of sodium hydride was suspended in 200 mL of ether, and thereto was slowly added dropwise at 0°C a solution of 12.4 g (140 mmol) 3-hydroxytetrahydrofuran dissolved in 50 mL of ether. 33.7 g (200 mmol) of trifluoromethanesulfonyl chloride was added dropwise the resulting solution and refluxed for 3 hours. After cooling, water was added to the resulting solution and an ether layer was separated. The ether was distilled off and the residue was distilled under reduced pressure to obtain 26.2 g (119 mmol) of 3-(trifluoromethanesulfonato)-tetrahydrofuran. The yield was 85%.

According to the present invention, a 3-aminomethyltetrahydrofuran derivative, which is useful as an intermediate for medicines and agrochemicals, can be obtained and provided in the synthesis of the medicines and agrochemicals.

## Claims

1. A process for producing a 3-aminomethyltetrahydrofuran derivative represented by the formula (2): 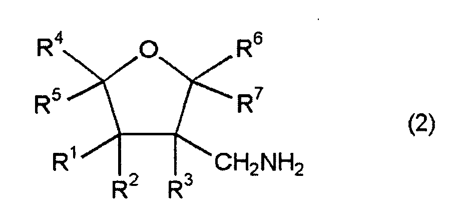 wherein R¹, R², R³, R⁴, R⁵, R⁶ and R⁷ may be the same or different from each other and each represent a hydrogen atom or a hydrocarbon group having 1 to 4 carbon atoms,
comprising:
reducing the cyano group of a 3-cyanotetrahydrofuran derivative represented by the formula (1): 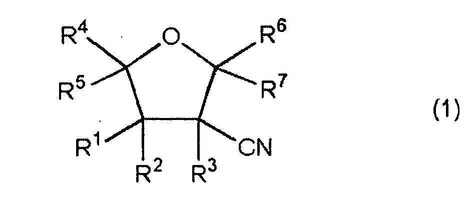
wherein R¹, R², R³, R⁴, R⁵, R⁶, and R⁷ may be the same or different from each other and each represent a hydrogen atom or a hydrocarbon group having 1 to 4 carbon atoms.

2. The process for producing a 3-aminomethyltetrahydrofuran derivative as set forth in claim 1, wherein the cyano group of the 3-cyanotetrahydrofuran derivative is reduced with hydrogen in the presence of ammonia and in the presence of a metal of Group 9 or 10 of the Periodic Table of the Elements or a metal compound thereof, as a catalyst.

3. The process as set forth in claim 2, wherein the catalyst is a metal such as cobalt or nickel or a metal compound thereof.

4. The process as set forth in claim 2 or 3, wherein the ammonia is aqueous ammonia.

5. The process as set forth in claim 4, wherein the reaction is carried out in the presence of 0.05 to 2 parts by weight of water relative to 1 part by weight of the 3-cyanotetrahydrofuran derivative.

6. A process for producing a 3-cyanotetrahydrofuran derivative represented by the formula (1),
comprising:
reacting a 3-halogenated or 3-alkylsulfonated or 3-arylsulfonated tetrahydrofuran derivative represented by the formula (3): 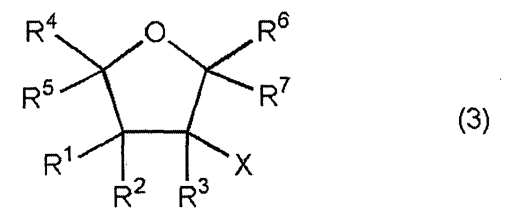
wherein R¹, R², R³, R⁴, R⁵, R⁶ and R⁷ may be the same or different from each other and each represent a hydrogen atom or a hydrocarbon group having 1 to 4 carbon atoms; and X represents a halogen atom or an alkylsulfonate group having 1 to 6 carbon atoms or arylsulfonate group having 6 to 12 carbon atoms, and an organic or inorganic cyano compound.

7. The process as set forth in claim 6, wherein the organic or inorganic cyano compound is alkali metal cyanide and,
the reaction is carried out using in the presence of the solvent having the permittivity of 20 F·m⁻¹ or more.

8. The process as set forth in claim 7, wherein the solvent having the permittivity of 20 F·m⁻¹ or more is an aprotic solvent.

9. The process as set forth in any one of claims 1 to 5, wherein the 3-cyanotetrahydrofuran derivative is obtained by the process as set forth in any one of claims 6 to 8.

10. The process as set forth in claim 9, wherein the 3-halogenated or 3-alkylsulfonated or 3-arylsulfonated tetrahydrofuran derivative represented by the formula (3a) is prepared from the malic acid derivative by the following first to third steps:
[First Step]
A -COOR⁸ group and a -COOR⁹ group of the malic acid derivative represented by the formula (4): 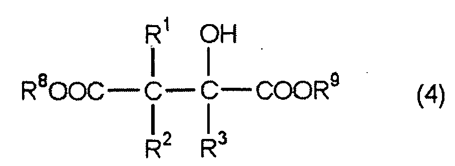 wherein R¹, R², R³, R⁸ and R⁹ may be the same or different from each other and each represent a hydrogen atom or a hydrocarbon group having 1 to 4 carbon atoms,
are reduced to prepare triols represented by the formula (5): 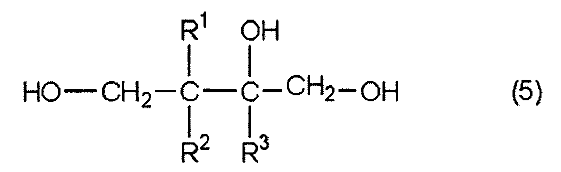 wherein R¹, R² and R³ may be the same or different from each other and each represent a hydrogen atom or a hydrocarbon group having 1 to 4 carbon atoms,
corresponding to the malic acid derivative used.
[Second Step]
The triols represented by the formula (5) obtained from the first step is subjected to an intramolecular dehydration reaction in the presence of an acid catalyst to prepare a 3-hydroxytetrahydrofuran derivative represented by the formula (6): 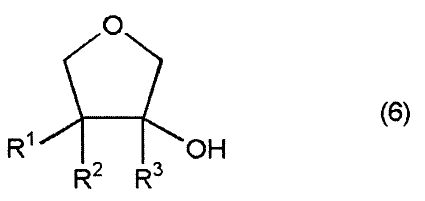 wherein R¹, R² and R³ may be the same or different from each other and each represent a hydrogen atom or a hydrocarbon group having 1 to 4 carbon atoms,
corresponding to the triols used.
[Third Step]
The 3-hydroxytetrahydrofuran derivative represented by the formula (6) obtained from the second step and a halogenating agent or alkyl or arylsulfonylating agent are reacted to halogenate or alkyl or arylsulfonate a hydroxyl group thus to prepare the 3-halogenated or 3-alkylsulfonated or 3-arylsulfonated tetrahydrofuran derivative represented by the formula (3a): 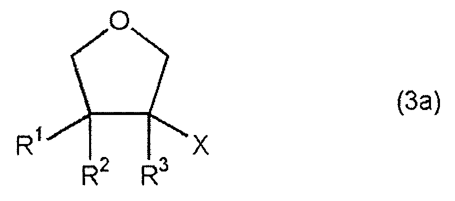 wherein R¹, R² and R³ may be the same or different from each other and each represent a hydrogen atom or a hydrocarbon group having 1 to 4 carbon atoms; and X represents a halogen atom, or an alkylsulfonate group having 1 to 6 carbon atoms or an arylsulfonate group having 6 to 12 carbon atoms.
